# EUROPEAN PATENT SPECIFICATION

(11) **EP 2 089 401 B1**
(45) Date of publication and mention of the grant of the patent: **25.07.2012**
(21) Application number: 07822469.8
(22) Date of filing: 09.11.2007
(51) Int. Cl.: C07F 9/10, A61K 31/661, A61P 35/00

(54) **USE OF TRI-SUBSTITUTED GLYCEROL COMPOUNDS FOR THE TREATMENT OF RADIATION INJURIES**
VERWENDUNG VON TRISUBSTITUIERTEN GLYCERINVERBINDUNGEN ZUR BEHANDLUNG VON STRAHLUNGSVERLETZUNGEN
UTILISATION DE COMPOSÉS DE GLYCÉROL TRISUBSTITUÉ POUR LE TRAITEMENT DE LÉSIONS RADIQUES

(30) Priority: 10.11.2006 US 858170 P
(43) Date of publication of application: 19.08.2009
(73) Proprietor: RadioProtect Alphaptose UG (haftungsbeschränkt) & Co. KG, 23701 Eutin (DE)
(72) Inventor: RICHTER, Wolfgang, 81243 München (DE); WEBER, Lutz, 82110 Germering (DE)
(74) Representative: Maiwald Patentanwalts GmbH
(86) International application number: PCT/EP2007/062181
(87) International publication number: WO 2008/055997

(56) References cited:
- WO-A-01/76357
- WO-A-2005/020935
- DE-A1- 19 822 509
- PINCHUK A N ET AL: "SYNTHESIS OF ALKYL GLYCEROPHOSPHOLIPIDS THROUGH 1-0-BENZYL-2-0-METHYL-rac-GLYCEROL" PHARMACEUTICAL CHEMISTRY JOURNAL, CONSULTANTS BUREAU, NAW YORK, NY, US, vol. 26, 1992, pages 174-176, XP008087544 ISSN: 0091-150X

## Description

The present invention relates to the use of a tri-substituted glycerol compound or a pharmaceutically acceptable salt thereof for the manufacture of a medicament for the prevention and/or treatment of radiation injuries. Furthermore, the invention relates to *in vitro* corresponding methods for preventing or treating of radiation damage or injury in one or more cells comprising contacting said cells with a medicament as defined in the invention.

Exposure to radiation (such as X-rays, gamma rays, and alpha-or beta- radiation) can cause damage to cells. This damage can result in cell death (e.g., through apoptosis), or can cause genetic changes in the cell, resulting in uncontrolled cell proliferation and as a consequence to the development and progression of tumors.

While, in general, exposure to such radiation is undesirable, the administration of carefully monitored doses of radiation is an accepted treatment for certain cancers such as leukemia, breast cancer, prostate cancer or colon cancer. By targeting the radiation to a tumor, cancer cells can be destroyed.

A frequent complication of radiotherapy is the irradiation of normal tissues surrounding the cancerous tissues. Such normal tissues are often damaged by the radiation, resulting in an undesired radiation injury to normal cells and tissues, which can have severe consequences for the affected patient.

Exposure to radiation can occur in several other ways, including exposure to normal background levels of radiation (such as cosmic rays or radiation due to naturally occurring isotopes present in the earth) or elevated environmental radiation (including occupational exposure of persons in medical facilities or nuclear power plants as well as exposure to X-rays during medical diagnosis). Another potential source of exposure to certain types of radiation is the accidental or intentional release of radioactive materials, for example, as the result of an accident or as a result of terrorist activity, e.g., as the result of a radiologic weapon such as a so-called "dirty bomb" (an explosive device intended to spread radioactive materials to contaminate an area).

The primary form of protection against radiation injury is avoidance of exposure to radiation. Shielding materials capable of preventing penetration of radiation into the body can be used when a source of radiation is known. For example, lead aprons can be used to block x-rays. Protective clothing can be used to prevent contamination of the body with radioactive materials, and decontamination procedures can be used to remove radioactive materials.

Treatment with radioprotective chemical compounds is an approach for preventing certain types of radiation damage, such as DNA damage due to free radicals (or other reactive species) produced by the radiation.

A widely used radioprotective agent is amifostine, an organic thiophosphate prodrug (2-[(3-aminopropyl)amino]ethanethiol dihydrogen phosphate) that is dephosphorylated *in vivo* by alkaline phosphatase to the active thiol metabolite (cf., for example, the US Patent 7,073,072 as well as the International patent Applications WO 02/092103 and WO 02/062350). The selective protection of non-malignant tissues is believed to be due to higher alkaline phosphatase activity, higher pH, and vascular permeation of normal tissues. Amifostine is used therapeutically *inter alia* to reduce the incidence of neutropenia-related fever, to decrease the cumulative nephrotoxicity associated with platinum-containing agents, and to reduce the incidence of xerostomia in patients undergoing radiotherapy for head and neck cancer. However, it has been shown that amifostine is only effective as radioprotectant when administered shortly prior to an exposure to radiation. An administration after exposure to radiation is without any therapeutic effect. Furthermore, the administration of amifostine or related thiol compounds is associated with pronounced adverse side effects such as systemic cytotoxicity as well as gastrointestinal incompatibilities like nausea and vomiting.

WO 2005/020935 A1 describes methods and compositions, wherein amifostine is administered with vitamine E, in particular in liposomes, in order to reduce the toxicity attributable to amifostine.

Another compound, 5-androstenediol, has been tested as a radiation protectant in preclinical animal studies. This compound is reported to improve survival in mice exposed to radiation, possibly by stimulating production of neutrophils and other immune-system cells and thus preventing infection, a significant cause of death in radiation-injured subjects. However, this compound is a salvaging measure and it does not counteract the pathogenic mechanism of radiation nor protect organs other than the hematopoietic system. It has not yet been approved for human use.

As promising these prophylactic radiation protection effects may appear, as modest are the results of efforts to find a therapy with chemical substances given after irradiation. There are attempts to treat the irradiation-damaged nucleic acids by substituting the DNA or RNA. The initial results of these investigations, however, are not encouraging to further develop a therapeutic concept. Thus, the only option remaining is to treat the indirect consequences of the cellular damages, for example the impact on bone marrow insufficiency that governs the clinical outcome after a high dose whole body irradiation. Secondary effects of bone marrow damages are infections that are caused by leukopenia and accompanied by fever, agranulocytosis, petechiae and profuse bleeding as a consequence of thrombocytopenia. These symptoms may cause death in severe cases. A therapy of these secondary effects of the bone marrow damages includes antibiotic treatment as well as a substitution therapy with the blood cells that are missing such as granulocytes and thrombocytes. The *ultima ratio* in cases of very high radiation burden would be bone marrow transplantation.

While level of intensive care for lethally irradiated victims might be very high, such therapy will be available only in few specialized hospitals for a limited number of patients. In case of a real nuclear catastrophe with probably hundreds of highly irradiated patients such demanding specialized treatment is not feasible. An intensive care therapy using bone marrow transplantation may rescue people with a whole body irradiation of 10 Gy (Gray). However, in case of untreated or insufficiently treated victims the lethal dose is reduced to 3-4 Gy. Thus, an appropriate therapeutic approach in such scenario would be a chemotherapy that is effective to lead to an increased prognosis of those mid-level lethally irradiated people.

Tri-substituted glycerol compounds belonging to the class of synthetic ether-linked alkyl-lysophospholipids might be candidate compounds for such radioprotective therapy. In preliminary analyses, certain alkyl-lysophospholipid analogs have been shown to have a beneficial effect to cells upon an exposure to low X-ray radiation in mice (Berdel, W. et al. (1983) Radiation Res. 94, 166-170). However, this study is silent with regard to a generalization to other types of radiation as well as concerning the radiation doses that can be treated. Furthermore, it has still to be unraveled whether such alkyl-lysophospholipid analogs are capable of both to prevent radiation damage or injury and to treat them.

Synthetic ether-linked alkyl-lysophospholipids are known to have an anti-cancerogenic activity (reviewed, e.g., by Arthur, G., and Bittman, R. (1998) Biochim. Biophys. Acta 1390, 85-102; Jendrossek, V., and Handrick, R. (2003) Curr. Med. Chem. Anti-Canc. Agents 3, 343-353; Mollinedo, F. et al. (2004) Curr. Med. Chem. 11, 3163-3184). 1-*O*-octadecyl-2-*O*-methyl-glycero-3-phosphocholine (also referred to as ET-18-OCH3, AP-121 or edelfosine) is considered to be the prototype of these lipids. 1-*O*-octadecyl-2-*O*-methyl-glycero-3-phosphocholine represents a synthetic analogue of the platelet activating factor (PAF; 1-O-alkyl-2-acetyl-sn-glycero-3-phosphocholine), a potent phospholipid activator and mediator of many leukocyte functions, including platelet aggregation, inflammation, and anaphylaxis. Unlike most conventional chemotherapeutic drugs, these synthetic ether lipids do not directly target cellular DNA but rather affect the plasma membrane lipid composition and/or interfere with various signal transduction pathways. Thus, their mode of action does not depend on the presence of particular cellular receptors or is it cell cycle-dependent.

Cancer chemotherapy generally aims to slow the growth of, or destroy, cancer cells while avoiding collateral damage to surrounding cells and tissues. Consequently, the most effective anticancer agents are those that are able to selectively target cancer cells while leaving normal cells relatively unaffected. Synthetic ether-lipids have been shown to exert such an effect (cf., for example, Magistrelli, A. et al. (1995) Drug. Metab. Dispos. 23, 113-118). Several mechanisms of action have been proposed for the toxicity of ether-lipids towards cancer cells, including the cells' lack of alkyl cleavage enzymes. The resultant inability to hydrolyze the ether-lipids leads to their intracellular accumulation and to consequent damage to cell membrane lipid organization. Other potential mechanisms of ether-lipid action include effects on levels of intracellular protein phosphorylation, and disruption of cellular lipid metabolism. Normal cells typically possess the means to avoid or overcome the potentially toxic effects of ether-lipids, while cancer cells do not.

Thus far, synthetic ether lipids have been used for the treatment of different types of tumors such as brain tumors or mamma carcinomas (cf., for example, the German Patent DE 2619686 as well as the International Patent Applications WO 99/59599 and WO 00/01392, respectively).

Although the anti-tumor activity of these synthetic ether lipids has been experimentally proven in several animal tumor models, their clinical use is often hampered by systemic cytotoxic effects including hemolysis, particularly in the gastrointestinal tract but also *inter alia* in lung, liver or kidney. In 10-20% of the patients treated with such water- and/or milk-based vehicles containing ether lipids severe gastrointestinal incompatibilities corresponding to WHO toxicity grades III or IV have been observed that are associated with nausea, vomiting, diarrhea or constipation (see, e.g., Drings, P. et al. (1992) Onkologie 15, 375-382).

Thus, aside from exerting the desired pharmaceutical efficacy there is a need for a medicament, which allows for an easy and convenient administration. In particular, there remains a need for a medicament that is simultaneously suitable for preventing radiation damage or injury prior to exposure to radiation and for ameliorating or treating radiation damage or injury once the exposure to radiation has occurred.

Accordingly, it is an object of the present invention to provide a medicament for the prevention and/or treatment of radiation damage or injury having such properties.

This object is achieved by the use of a tri-substituted glycerol compound having the features of independent claim 1 for the manufacture of a corresponding medicament. Some of the preferred embodiments of the present invention are defined by the subject matter of the dependent claims.

Surprisingly, it has been found that tri-substituted glycerol compounds such as 1-O-octadecyl-2-O-methyl-glycero-3-phosphocholine that are known as anti-cancerogenic agents are also exerting a radioprotective effect on cells and tissues, which allows for an efficient prevention and/or treatment of radiation damage or injury in response to different types of radiation. The inventive medicament provides the desired efficacy, can be conveniently administered to a patient, and does not show adverse side effects.

In the context of the present invention any numerical value indicated is typically associated with an interval of accuracy that the person skilled in the art will understand to still ensure the technical effect of the feature in question. As used herein, the deviation from the indicated numerical value is in the range of ± 10%, and preferably of ± 5%.

In a first aspect, the present invention relates to the use of a tri-substituted glycerol compound according to formula (I) 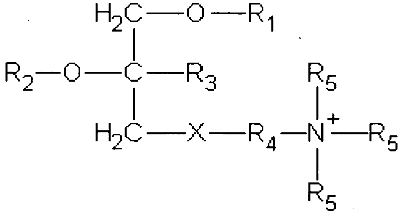 or an enantiomer or diastereomer or a pharmaceutically acceptable salt thereof and at least one pharmaceutically acceptable excipient for the manufacture of a medicament for the prevention and/or treatment of radiation damage or injury, wherein
X is selected from the group consisting of phosphate and sulfate;
R₁ is selected from the group consisting of C₁₆- C₂₀ alkyl;
R₂ is selected from the group consisting of C₁-C₃ alkyl and C₁-C₃ hydroxyalkyl;
R₃ is selected from the group consisting of hydrogen and C₁-C₃ alkyl;
R₄ is selected from the group consisting of C₁-C₃ alkyl and C₃-C₆ cycloalkyl;
and
R₅ is selected from the group consisting of hydrogen and methyl.

The tri-substituted glycerol compound may be present in amorphous or in crystalline form. The term "amorphous", as used herein, refers to a solid in which there is no long-range order of the positions of the atoms, i.e. a non-crystalline material. In preferred embodiments of the invention, the tri-substituted glycerol compound is present in crystalline form.

The terms "Cₙ alkyl", "Cₙ hydroxyalkyl", and "Cₙ cycloalkyl", as used herein, denote an alkyl group, a hydroxyalkyl group or a cycloalkyl group having n carbon atoms, respectively. For example, the term "C₁₈ alkyl" refers to an alkyl group having 18 carbon atoms. The alkyl groups or hydroxyalkyl groups according to the invention may be straight or branched.

The tri-substituted glycerol compounds of formula (I) have one or more asymmetric centers and thus they can exist as enantiomers or diastereomers. Thus, the medicament as defined in the present invention may comprise either one or more separate individual isomers (such as the L form and the D form) or mixtures of isomers, preferably racemic mixtures.

In some embodiments of the invention, the tri-substituted glycerol compounds of formula (I) are present in the medicament as pharmaceutically acceptable salts. Such salts may comprise any pharmaceutically acceptable anion "neutralizing" the positive charge of the nitrogen (e.g. chloride, bromide or iodide) or any pharmaceutically acceptable cation "neutralizing" the negative charge of the phosphate or sulfate moiety (e.g. sodium or potassium cations).

In a particular preferred embodiment of the present invention, the pharmaceutical solid dosage form comprises a tri-substituted glycerol compound according to formula (I), wherein X is phosphate, R₁ is -(CH₂)₁₇-CH₃, R₂ is CH₃, R₃ is H, R₄ is - (CH₂)₂-, and R₅ is CH₃.

The medicament according to the present invention may be any pharmaceutical dosage form that is therapeutically effective. Examples of such pharmaceutical dosage forms include *inter alia* tablets, pills, capsules, suspensions, emulsions, injection or infusion solutions, tinctures, powders and the like.

The medicaments used in the present invention comprise at least one pharmaceutically acceptable excipient. The term "pharmaceutically acceptable excipient", as used herein denotes any substance used for the preparation of pharmaceutical dosage forms such as coating materials, film-forming materials, fillers, disintegrating agents, release-modifying materials, carrier materials, diluents, binding agents and other adjuvants, all of them well known in the art (cf. the references cited below). Preferably, the excipient used in the invention comprises at least one filler, at least one binder, at least one disintegrating agent, at least one flowability-controlling agent, and at least one lubricant.

The medicament may be administered via any parenteral or non-parenteral route. Parenteral application methods comprise, for example, intracutaneous, subcutaneous, intramuscular or intravenous injection and infusion techniques. Non-parenteral delivery modes include, for instance, oral or topical administration. Furthermore, the medicament may be administered locally or systemically.

Preferably, the medicament employed in the present invention is a pharmaceutical dosage form suitable for oral application. Particularly preferably, the dosage form is a solid dosage form. Examples of such dosage forms include *inter alia* tablets, pills, capsules, granulates, pellets, powders, multi-particulate formulations (e.g., beads, granules or crystals), and dragees. The unit doses of multi-particulates may be incorporated into a pharmaceutical solid dosage form, e.g. via compression or shaping into tablets or by placing a requisite amount inside a gelatin capsule.

All these solid dosage forms for oral application as well as methods for their preparation are well established in the art (see, e.g., Gennaro, A.L. and Gennaro, A.R. (2000) Remington: The Science and Practice of Pharmacy, 20th Ed., Lippincott Williams & Wilkins, Philadelphia, PA; Ritschel, W.A. & Bauer-Brandl, A. (2002) Die Tablette: Handbuch der Entwicklung, Herstellung und Qualitätssicherung. Editio-Cantor Verlag, Aulendorf, Germany; Crowder, T.M. et al. (2003) A Guide to Pharmaceutical Particulate Science. Interpharm/CRC, Boca Raton, FL; Stricker, H. (2003) Arzneiformenentwicklung, Springer Verlag, Berlin, Germany; Niazi, S.K. (2004) Handbook of Pharmaceutical Manufacturing Formulations, CRC Press, Boca Raton, FL).

In preferred embodiments of the invention, the pharmaceutical solid dosage form is selected from the group consisting of tablets, pills, capsules, and granules, with tablets being particularly preferred.

Preferably, the solid dosage form is an enteric dosage form. That is, the dosage form remains stable in the stomach, i.e. in an acidic environment, with pH values in the range of ≤ 2.5. This may be achieved by providing a solid dosage form comprising a film coating. For example, the inventive dosage form may be in the form of a so-called film tablet.

Methods for the preparation of film coated dosage forms are also well established in the art (see, e.g., the references cited above). Furthermore, the skilled artisan also knows how to provide film coatings with specific properties, like enteric coatings, film coating which dissolve upon contact with body fluids, controlled release coatings, taste-masking coatings or disintegrating coatings. In a particularly preferred embodiment, the solid dosage form of the invention comprises an enteric coating.

According to the present invention, it is to be understood that the tri-substituted glycerol compound is present in the medicament in any amount being effective to achieve the desired pharmacological effect when administered to a patient. Effective amounts are generally chosen in accordance with a number of factors, e.g., the age, size and general condition of the patient and the medical condition being treated, and determined by a variety of means, for example, dose ranging trials, well known to, and readily practiced by persons of ordinary skill in art given the teachings of this invention.

Typically, in a medicament as defined in the present invention the amount of the tri-substituted glycerol compound according to formula (I) is less than 400 mg, preferably it is in the range of 30 to 250 mg, and most preferably it is in the range of 50 to 150 mg. In particularly preferred embodiments of the invention, the amount of the tri-substituted glycerol compound according to formula (I) is 75 mg and 100 mg, respectively.

The daily dosage of the tri-substituted glycerol compound administered to a patient is less than 1200 mg, typically less than 900 mg, preferably in the range of 30 to 600 mg, more preferably in the range of 40 to 400 mg, and most preferably in the range of 50 to 350 mg. In specific embodiments, the daily dosage is 75, 100, 150, 200, 225, and 300 mg. Preferably, the daily dosage of the tri-substituted glycerol compound is administered as a single dose such as in form of one up to four tablets or capsules. However, it may also be possible to administer the compound in multiple doses such as two or three individual doses administered during the day, e.g. in the morning, at noon, and at night.

The medicament according to the invention may be used for the prevention and/or treatment of radiation damage or injury individually or in combination with at least one other medicament comprising at least one additional active ingredient. That is, it is also within the scope of the present invention to use a medicament comprising a tri-substituted glycerol compound defined in the claims together with at least one other medicament comprising one or more different active ingredients such as chemotherapeutics or monoclonal antibodies.

The term "radiation damage or injury", as used herein, refers to any negative or adverse effect an exposure to radiation - independent of the radiation dose applied and the time of exposure, respectively - may exert on cells, tissues, organs or organisms resulting in uncontrolled cell proliferation and/or differentiation and as a consequence to the development and progression of tumors. Examples of such radiation damages or injuries include *inter alia* genetic changes in the cell (e.g., DNA and/or RNA mutations, DNA and/or RNA decay, chromosomal aberrations) as well as cell death (e.g., programmed cell death/apoptosis).

In some embodiments of the invention the radiation damage or injury is caused by ionizing radiation. The term "ionizing" radiation" as used herein, denotes either particle radiation or electromagnetic radiation in which an individual particle/photon carries enough energy to ionize an atom or molecule by completely removing an electron from its orbit. If the individual particles do not carry this amount of energy, it is essentially impossible for even a large flood of particles to cause ionization. These ionizations, if enough occur, can be very destructive to living tissue. Examples of particle radiation that are ionizing may be energetic electrons, neutrons, atomic ions or photons. Electromagnetic radiation can cause ionization if the energy per photon, or frequency, is high enough, and thus the wavelength is short enough. The amount of energy required varies between molecules being ionized.

Preferably, the ionizing radiation is selected from the group consisting of neutron radiation, alpha radiation, beta radiation, gamma radiation, and X-rays.

Neutron radiation is often called indirectly ionizing radiation. It does not ionize atoms in the same way protons, photons, and electrons do because neutrons have no charge. However, neutron interactions are largely ionizing, for example when neutron absorption results in gamma emission and the gamma subsequently removes an electron from an atom, or a nucleus recoiling from a neutron interaction is ionized and causes more traditional subsequent ionization in other atoms. Because neutrons are uncharged, they are more penetrating than alpha radiation (helium nuclei) and beta radiation (electrons or positrons). In some cases they are more penetrating than gamma radiation (electromagnetic radiation), which is impeded in materials of high atomic number.

X-rays are a form of electromagnetic radiation with a wavelength in the range of 10 to 0.01 nm, corresponding to frequencies in the range 30 to 30 000 PHz (10¹⁵ Hz). X-rays are primarily used for diagnostic radiography and cristallography. X-rays are a form of ionizing radiation.

Within the scope of the present invention, the radiation damage or injury to be prevented and/or treated may be the result of an exposure to naturally occurring or to artificial radiation. Exposure to radiation, within the meaning of the invention, can occur in several other ways, including exposure to normal background levels of radiation (such as cosmic rays or radiation due to naturally-occurring isotopes present in the earth) or elevated environmental radiation (including occupational exposure of persons in medical facilities or nuclear power plants as well as exposure to X-rays during medical diagnosis, e.g. computer tomography). Another potential source of exposure to certain types of radiation is the accidental or intentional release of radioactive materials, for example, as the result of an accident or as a result of terrorist activity, e.g., as the result of a radiologic weapon such as a so-called "dirty bomb" (an explosive device intended to spread radioactive materials to contaminate an area).

In preferred embodiments of the invention, the radiation damage or injury is associated with cancer therapy, i.e. is the result of cancer radiotherapy. In a special embodiment of the invention, the radiation damage or injury is associated with bone marrow transplantation during cancer therapy.

In a second aspect, the present invention relates to a tri-substituted glycerol compound, as defined, herein for the prevention and/or treatment of radiation damage or injury. In preferred embodiments, the radiation damage or injury is associated with cancer therapy or with bone marrow transplantation during cancer therapy.

In a third aspect, the present disclosure relates to a corresponding method for the prevention and/or the treatment of radiation damage or injury, wherein the method comprises administering to a patient a medicament as defined in the invention.

As outlined above, the medicament according to the present invention may be administered via any parenteral or non-parenteral route. Preferably, the medicament is administered orally. Furthermore, preferably, the medicament may be administered as a single dose such as in form of one tablet or capsule per day. However, it may also be possible to administer the medicament in multiple doses such as two or three individual doses administered during the day. For the prevention of radiation damage or injury the medicament is preferably administered prior to an exposure to radiation. However, it is also possible to administer the medicament during and/or after an exposure to radiation.

In a forth aspect, the invention relates to *in vitro* methods for preventing radiation damage or injury and for treating radiation damage or injury in one or more cells, respectively, each of them comprising contacting the one or more cells with a medicament as defined in the invention. In the first method, the cells are contacted with the medicament prior to an exposure to radiation.

Preferably, the one or more cells are non-cancerous cells (i.e. non-tumorgenic control cells) such as bone marrow cells.

The invention is further described by the following figures and examples, which are solely for the purpose of illustrating specific embodiments of this invention, and are not to be construed as limiting the scope of the invention in any way.

Materials used in tests below are either commercially available or easily prepared from commercially available materials by those skilled in the art.

### FIGURES

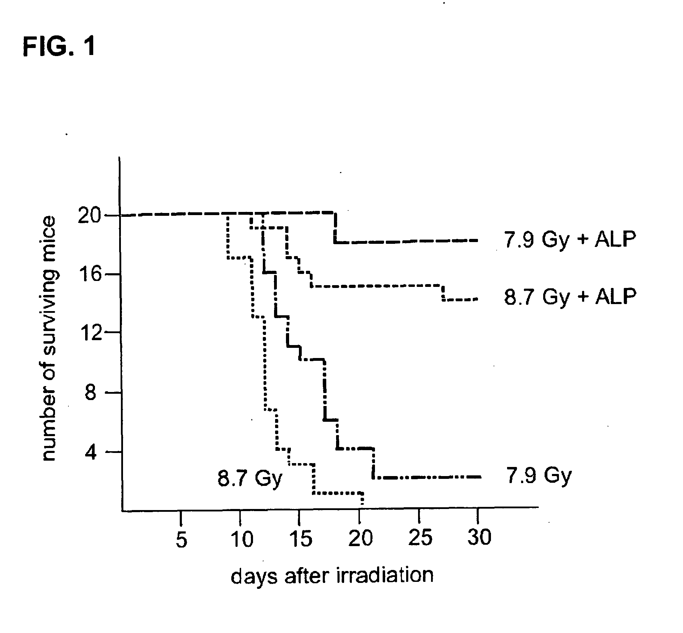

- **Figure 1**: depicts the radioprotective effect of ET 18-OCH3 on the survival rate of mice. ET 18-OCH3 was administered as a single dose of 50 mg/kg body weight to each of 40 mice (20 control mice, 20 test mice). 24 hours later, the 20 test mice were exposed to a gamma radiation dose of 7.9 Gy (Gray) and 8.7 Gy, respectively. The survival rates were monitored for 30 days.

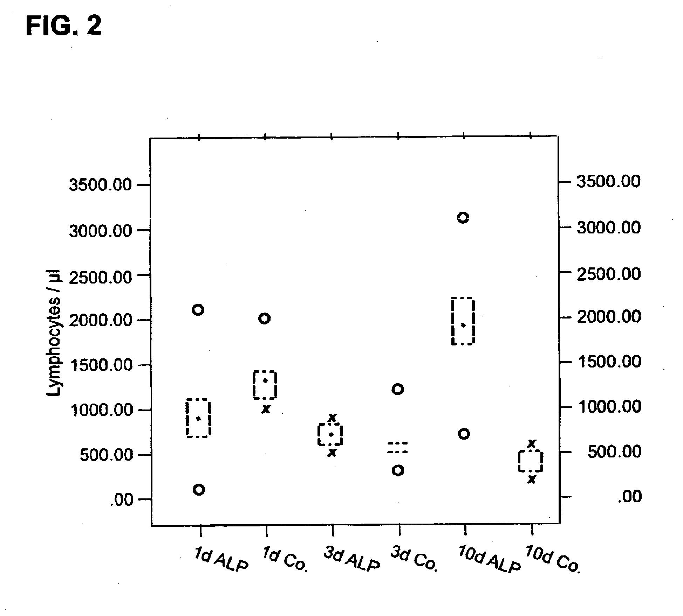

- **Figure 2**: depicts the radioprotective effect of ET 18-OCH3 (referred to as "ALP") on the lymphocyte numbers of mice. Mice were treated with a single dose 70 mg/kg ET 18-OCH3 (administered subcutaneously) 24 hours before exposure to neutron radiation (radiation dose 2.0 Gy). The number of lymphocytes per µl blood of untreated (irradiated) control mice and treated (test) mice was determined at days 1, 3, and 10 after irradiation.

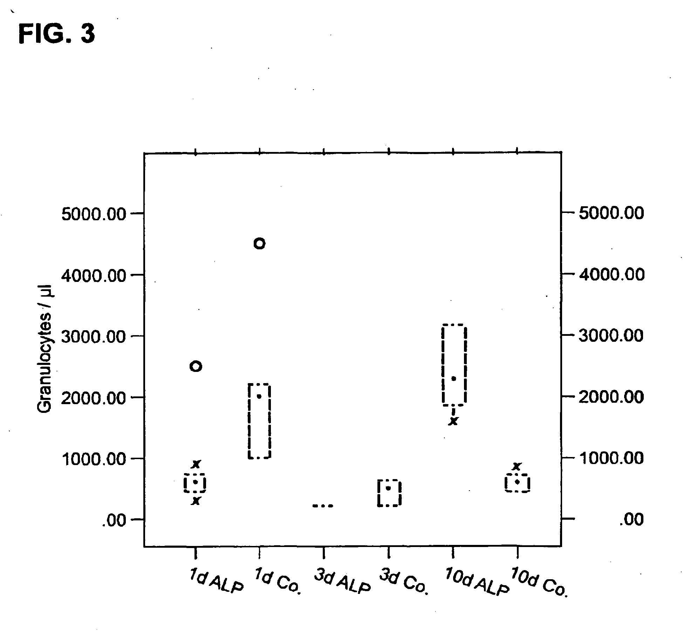

- **Figure 3**: depicts the radioprotective effect of ET 18-OCH3 (referred to as "ALP") on the granulocyte numbers of mice. The experiment was performed in an analogous manner as described in Fig. 2.

### EXAMPLES

The efficacy of 1-*O*-octadecyl-2-*O*-methyl-glycero-3-phosphocholine (in the following referred to as "ET18-OCH3") as a radioprotectant in the treatment of an acute radiation injury that is caused by (mid)lethal radiation doses was analyzed by determining its influence on the survival rate of mice and on the hematological syndrome of the radiation injury in response to different types of radiation.

### Example 1: Efficacy of ET18-OCH3 in response to X-rays

A single dose of 25mg/kg ET18-OCH3 was administered at a time intravenously to 25 mice 12 hours after exposure to an X-ray dose of 650 cGy (centiGray). Only one of the 25 treated mice died, as compared to 6 of the 25 mice in the control group.

After an intravenous administration of 25mg/kg ET18-OCH3 6 hours and 12 hours after X-ray irradiation, respectively, the following results were obtained (Table 1). The chi-square distribution is given for all positive values. The chi-square test was used for comparing the results of the treated versus the control animals.

**Table 1**: Survival rates of X-ray irradiated mice after administration of 2 x 25 mg/kg ET18-OCH3 6 hours and 12 hours after irradiation, respectively.

| **X-ray Dose (cGy)** | **Death/Survival control** | **Death/Survival treated** | **Chi²** | **p** |
|---|---|---|---|---|
| 650 | 4/26 | 2/28 | 0.74 | 0.402 |
| 700 | 19/11 | 3/25 | 17.03 | < 0.001 |
| 750 | 27/3 | 14/16 | 13.02 | < 0.001 |
| 800 | 30/0 | 22/8 | 9.23 | 0.002 |

The LD_{50/30} X-ray dose was increased from 688.1 ± 38.6 to 749 ± 37.1 cGy for the treated *versus* the untreated controls animals. X-ray mediated lethality was delayed in the treated mice, as compared to the control mice.

After an oral administration of 25mg/kg ET18-OCH3 6 hours and 24 hours after exposure to a X-ray dose of 700 cGy X-ray irradiation, respectively, 2 of 25 treated animals died, as compared to 7 of 25 of the control animals.

### Example 2: Efficacy of ET 18-OCH3 in response to neutron radiation

Mice were irradiated using a van de Graaf generator with of 3-8 MeV neutrons. The radiation doses used in the following analyses were 400, 410, and 420 cGy for each third of the treated and the control mice, respectively. A single dose of 50 mg/kg ET18-OCH3 was administered to half of the mice by subcutaneous injection. The results obtained are shown in Table 2.

**Table 2**: Survival rates of mice after exposure to 400-420 cGy neutron radiation and subcutaneous administration of 50 mg/kg ET18-OCH3 at various time points. The chi-square test was used to compare the treated versus the control animals.

| **Number** | **Treatment** | **Survival/Death** | **Comp.** | **Chi²** | **p** |
|---|---|---|---|---|---|
| 1 | - | 8 (38%) / 13 (62%) | | | |
| 2 | ET 18-OCH3, 6 h | 12 (52%) / 11 (48%) | 1 vs. 2 | 0.88 | 0.382 |
| 3 | - | 8 (36%) / 14 (64%) | | | |
| 4 | ET18-OCH3, 2 h | 13 (54%) / 11 (46%) | 3 vs. 4 | 1.46 | 0.226 |
| 5 | | 6 (29%) / 15 (71%) | | | |
| 6 | ET 18-OCH3, 30 min | 18 (75%) / 6 (25%) | 5 vs. 6 | 9.79 | 0.002 |

From these results, it can be concluded that a treatment with a single dose of ET18-OCH3 should be carried out at the earliest possible time point after neutron irradiation. As the therapy experiments were carried out with an irradiation dose range, in which the hematological syndrome of the radiation injury predominates, the evaluation of the therapeutic effect of the drug on the blood forming (hematopoetic) system is important. From Table 3 it becomes apparent that ET18-OCH3 significantly increases the concentration of leukocytes and granulocytes in the peripheral blood during the time period when the irradiated mice died (day 10-15 after irradiation).

**Table 3**: Change in cell numbers in the peripheral blood of mice 7 and 14 days after exposure to a neutron irradiation of 300 cGy and subcutaneous administration of 50 mg/kg ET18-OCH3 2 hours and 30 min after irradiation, respectively. Given is the **median**/interquartile distance for various types of cells/µl; for thrombocytes the values are given in 1000 cells/µl. The Wilcoxon-Mann-Whitney U-test was used for comparison of controls versus treated animals (p-values).

| **Cell type** | **Time (days)** | **ET 18-OCH3 2h** | **Control 2h** | **p** | **ET 18-OCH3 30 min** | **Control 30 min** | **p** |
|---|---|---|---|---|---|---|---|
| Leukocytes | 7 | **675**/413 | **550**/200 | 0.07 | **975**/450 | **750**/450 | 0.38 |
| | 14 | **9975**/5863 | **4400**/9200 | 0.013 | **8675**/11827 | **3350**/1900 | 0.029 |
| Lymphocytes | 7 | **446**/132 | **399**/9200 | >0.4 | **684**/255 | **641**/264 | >0.4 |
| | 14 | **5101**/3765 | **2801**/3266 | 0.013 | **7439**/6942 | **1915**/1257 | 0.017 |
| Granulocytes | 7 | **240**/304 | **125**/132 | 0.034 | **209**/202 | **112**/264 | 0.1 |
| | 14 | **4189**/2282 | **1542**/3300 | 0.007 | **3063**/3121 | **854**/1089 | 0.011 |
| Thrombocytes | 7 | **900**/315 | **934**/273 | >0.4 | **698**/427 | **660**/50 | >0.4 |
| | 14 | **987**/464 | **979**/290 | >0:4 | **533**/263 | **580**/320 | >0.4 |
| Hematokrit | 7 | **42.5**/9.0 | **43.0**/5.0 | >0.4 | **40.0**/5.5 | **37.0**/8.8 | >0.4 |
| | 14 | **37.0**/8.5 | **31.0**/4.0 | 0.008 | **32.5**/5.0 | **28.0**/14.0 | 0.128 |

### Example 3: Efficacy of ET 18-OCH3 before applying gamma radiation

The effect of a prophylactic treatment with ET 18-OCH3 on the survival rate of gamma-radiated mice is summarized in Table 4.

**Table 4:** Survival rate of mice after gamma irradiation and administration of a single dose of ET18-OCH3 at various time points before exposure to irradiation or after radiation (a.r.), respectively. The chi-square test was used to compare the treated versus the control animals.

| **Dose (cGy)** | **Treatment ET18-OCH3** | **Survival** | **Death** | **Chi²** | **p** |
|---|---|---|---|---|---|
| 750 | - | 11 (55%) | 9 (45%) | | |
| 750 | 30mg/kg; a.r. | 17 (94%) | 1 (6%) | 7.60 | 0.006 |
| 775 | - | 0 | 20 (100%) | | |
| 775 | 40mg/kg; a.r. | 8 (40%) | 12 (60%) | 10.00 | 0.002 |
| 800 | - | 0 | 35(100%) | | |
| 800 | 40mg/kg; a.r. | 25 (71%) | 10 (29%) | 3889 | <0.001 |
| 775 | 50mg/kg; 2h | 15 (75%) | 5 (25%) | | |
| 800 | 50mg/kg; 2h | 17 (85%) | 3 (15%) | | |
| 750 | - | 10 (50%) | 10 (50%) | | |
| 750 | 50mg/kg; 1d | 20 (100%) | 0 | 13.33 | <0.001 |
| 775 | 50mg/kg; 1d | 18 (90%) | 2 (10%) | | |
| 790 | - | 2 (10%) | 18 (90%) | | |
| 790 | 50mg/kg; 1d | 18 (90%) | 2 (10%) | 25.60 | <0.001 |
| 830 | - | 1 (5%) | 19 (95%) | | |
| 830 | 50mg/kg; 1d | 17 (85%) | 3 (15%) | 25.85 | <0.001 |
| 870 | - | 0 | 20 (100%) | | |
| 870 | 50mg/kg; 1d | 13 (65%) | 7 (35%) | 19.26 | <0.001 |

Thus, a treatment of the mice with ET18-OCH3 resulted in a significant radioprotective effect that was similar when ET 18-OCH3 was given either 24 hours or shortly before irradiation. This is in contrast to the effect of conventional radioprotectants that is only apparent when the radioprotectant is given shortly before administration. When administering ET 18-OCH3 one day before irradiation, the LD_{50/30} gamma radiation dose increased from 7.47 Gy (untreated irradiated controls) to 8.98 Gy (treated irradiated mice) (as determined by a probit analysis). The effect of ET18-OCH3 on the survival rate of mice at two different radiation doses in depicted in Fig. 1

### Example 4: Efficacy of ET18-OCH3 before applying neutron radiation

The effect of ET 18-OCH3 before applying different doses of neutron radiation on the survival rates of mice are summarized in Table 5.

**Table 5**: Effect of ET18-OCH3 administered subcutaneously 1 day before neutron irradiation. The chi-square test was used to compare the treated versus the control animals.

| **Dosis (cGy)** | **Treatment** | **Survival** | **Death** | **Chi²** | **p** |
|---|---|---|---|---|---|
| 425 | - | 0 | 15 (100%) | | |
| 425 | 50mg/kg | 4 (27%) | 11 (73%) | 4.61 | 0.032 |
| 450 | - | 8 (28%) | 21 (72%) | | |
| 450 | 50mg/kg | 22 (73%) | 8 (26%) | 12.35 | <0.001 |
| 475 | - | 0 | 20 (100%) | | |
| 475 | 50mg/kg | 6 (24% | 19 (76%) | 5.54 | 0.019 |
| 425 | - | 0 | 15 (100%) | | |
| 425 | 70mg/kg | 5 (33%) | 10 (67%) | 6.00 | 0.014 |
| 450 | - | 1 (7%) | 14 (93%) | | |
| 450 | 70mg/kg | 3 (20%) | 12 (80%) | 1.15 | 0.283 |

### Example 5: Effect of ET18-OCH3 on the white blood cell count (hemogram)

After administration of 50 mg/kg and 70 mg/kg ET-18-OCH3, respectively, and exposure to neutron irradiation, the reduction of leukocyte numbers (both of lymphocytes and granulocytes) was slightly increased, as compared to untreated controls (not statistically significant; 5 experiments, n=90).

However, starting at day 3 after irradiation, the ET-18-OCH3 treatment resulted in an increase in leukocyte numbers (both lymphocytes and granulocytes), which became significant at day 7 (4 experiments; n=30, p<0.003). No statistically significant difference could be observed between the administration of 50 mg/kg and 70 mg/kg ET18-OCH3. The radioprotective effect of ET18-OCH3 on lymphocyte and granulocyte numbers in mice is shown in Fig. 2 and Fig. 3, respectively.

Despite a significant increase caused by the administration of ET18-OCH3, at midlethal irradiation doses the number of lymphocyte remains at a low level, which is average about one tenth of the normal value, whereas the number of leukocyte of the treated animals shows a much higher variation than that of the controls. Thus, at day 7 to 10 after irradiation the treated animals can be classified into two groups, in one of which the number of leukocytes is almost unchanged, as compared to the control group. These mice normally die at day 10 to14 after irradiation. The respective portions of leukocyte fractions was inconsistent, however, in general both the numbers leukocytes and granulocytes were significantly increased.

The present invention illustratively described herein may suitably be practiced in the absence of any element or elements, limitation or limitations, not specifically disclosed herein. Thus, for example, the terms "comprising", "including", "containing", etc. shall be read expansively and without limitation. Additionally, the terms and expressions employed herein have been used as terms of description and not of limitation, and there is no intention in the use of such terms and expressions of excluding any equivalents of the features shown and described or portions thereof, but it is recognized that various modifications are possible within the scope of the invention claimed. Thus, it should be understood that although the present invention has been specifically disclosed by preferred embodiments and optional features, modifications and variations of the inventions embodied therein herein disclosed may be resorted to by those skilled in the art, and that such modifications and variations are considered to be within the scope of this invention.

Other embodiments are within the following claims. In addition, where features or aspects of the invention are described in terms of Markush groups, those skilled in the art will recognize that the invention is also thereby described in terms of any individual member or subgroup of members of the Markush group.

## Claims

1. Use of a tri-substituted glycerol compound according to formula (I) 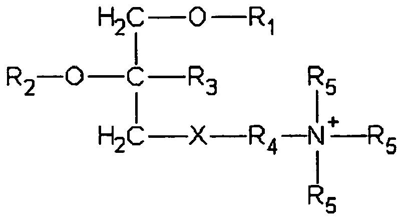 or an enantiomer or diastereomer or a pharmaceutically acceptable salt thereof and at least one pharmaceutically acceptable excipient for the manufacture of a medicament for the prevention and/or treatment of radiation damage or injury, wherein
X is selected from the group consisting of phosphate and sulfate;
R₁ is selected from the group consisting of C₁₆- C₂₀ alkyl;
R₂ is selected from the group consisting of C₁-C₃ alkyl and C₁-C₃ hydroxyalkyl;
R₃ is selected from the group consisting of hydrogen and C₁-C₃ alkyl;
R₄ is selected from the group consisting of C₁-C₃ alkyl and C₃-C₆ cycloalkyl; and
R₅ is selected from the group consisting of hydrogen and methyl.

2. The use according to claim 1, wherein X is phosphate, R₁ is -(CH₂)₁₇-CH₃, R₂ is CH₃, R₃ is H, R₄ is -(CH₂)₂-, and R₅ is CH₃.

3. The use according to claim 1 or 2, wherein the medicament is a dosage form for oral administration.

4. The use according to claim 3, wherein the dosage form is a solid dosage form.

5. The use according to claim 4, wherein the dosage form is selected from the group consisting of tablets, pills, capsules, and granules.

6. The use according to any of claims 1 to 5, wherein the amount of the tri-substituted glycerol compound in the medicament is in the range of 30 to 250 mg, preferably in the range of 50 to 150 mg.

7. The use according to any of claims 1 to 6, wherein the daily dosage of the tri-substituted glycerol compound is in the range of 50 to 350 mg.

8. The use according to any of claims 1 to 7, wherein the radiation damage or injury is caused by ionizing radiation, the ionizing radiation being preferably selected from the group consisting of neutron radiation, alpha radiation, beta radiation, gamma rays, and X-rays.

9. The use according to any of claims 1 to 8, wherein the radiation damage or injury is associated with cancer therapy, preferably with bone marrow transplantation during cancer therapy.

10. Tri-substituted glycerol compound as defined in any of claims 1 to 9 for use in the prevention and/or treatment of radiation damage or injury.

11. The tri-substituted glycerol compound according to claim 10, wherein the radiation damage or injury is associated with cancer therapy or with bone marrow transplantation during cancer therapy.

12. *In vitro* method for preventing radiation damage or injury in one or more cells, comprising: contacting the one or more cells prior to an exposure to radiation with a medicament as defined in any of claims 1 to 9.

13. *In vitro* method for treating radiation damage or injury in one or more cells, comprising: contacting the one or more cells with a medicament as defined in any of claims 1 to 9.

14. The *in vitro* method according to claim 12 or 13, wherein the one or more cells are non-cancerous cells, preferably bone marrow cells.

## Patentansprüche

1. Verwendung einer trisubstituierten Glycerolverbindung gemäß der Formel (I) 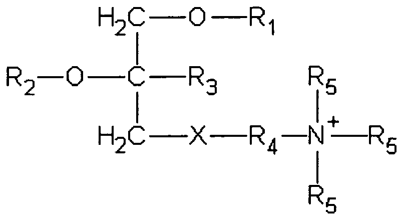 oder eines Enantiomers oder Diastereomers oder eines pharmazeutisch akzeptablen Salzes davon und mindestens eines pharmazeutisch akzeptablen Hilfsstoffs zur Herstellung eines Medikaments zur Vorbeugung und/oder Behandlung von Strahlenschäden oder durch Strahlung veranlasster Verletzung, wobei
X aus der Gruppe bestehend aus Phosphat und Sulfat ausgewählt ist;
R₁ aus der Gruppe bestehend aus C₁₆ -C₂₀ -Alkyl ausgewählt ist;
R₂ aus der Gruppe bestehend aus C₁-C₃-Alkyl und C₁-C₃-Hydroxyalkyl ausgewählt ist;
R₃ aus der Gruppe bestehend aus Wasserstoff und C₁-C₃-Alkyl ausgewählt ist;
R₄ aus der Gruppe bestehend aus C₁-C₃-Alkyl und C₃-C₆ -Cycloalkyl ausgewählt ist; und
R₅ aus der Gruppe bestehend aus Wasserstoff und Methyl ausgewählt ist.

2. Verwendung gemäß Anspruch 1, wobei X Phosphat ist,
R₁ -(CH₂)₁₇-CH₃ ist, R₂ CH₃ ist, R₃ H ist, R₄ -(CH₂)₂- ist, und R₅ CH₃ ist.

3. Verwendung gemäß Anspruch 1 oder 2, wobei das Medikament eine Verabreichungsform für orale Verabreichung ist.

4. Verwendung gemäß Anspruch 3, wobei die Verabreichungsform eine feste Verabreichungsform ist.

5. Verwendung gemäß Anspruch 4, wobei die Verabreichungsform aus der Gruppe bestehend aus Tabletten, Pillen, Kapseln und Granulat ausgewählt ist.

6. Verwendung gemäß einem der Ansprüche 1 bis 5, wobei die Menge der trisubstituierten Glycerolverbindung in dem Medikament im Bereich von 30 bis 250 mg, bevorzugt im Bereich von 50 bis 150 mg, liegt.

7. Verwendung gemäß einem der Ansprüche 1 bis 6, wobei die tägliche Dosierung der trisubstituierten Glycerolverbindung im Bereich von 50 bis 350 mg liegt.

8. Verwendung gemäß einem der Ansprüche 1 bis 7, wobei die Strahlenschäden oder die durch Strahlung veranlasste Verletzung durch ionisierende Strahlung verursacht wird, wobei die ionisierende Strahlung bevorzugt aus der Gruppe bestehend aus Neutronenstrahlung, Alphastrahlung, Betastrahlung, Gammastrahlen und Röntgenstrahlen ausgewählt ist.

9. Verwendung gemäß einem der Ansprüche 1 bis 8, wobei die Strahlenschäden oder die durch Strahlung veranlasste Verletzung mit Krebstherapie, bevorzugt mit Knochenmarks-Transplantation während der Krebstherapie, assoziiert ist.

10. Trisubstituierte Glycerolverbindung, wie in einem der Ansprüche 1 bis 9 definiert, zur Vorbeugung und/oder Behandlung von Strahlenschäden oder durch Strahlung veranlasster Verletzung.

11. Trisubstituierte Glycerolverbindung gemäß Anspruch 10, wobei die Strahlenschäden oder die durch Strahlung veranlasster Verletzung mit Krebstherapie oder mit Knochenmarks-Transplantation während einer Krebstherapie assoziiert ist.

12. *In vitro*-Verfahren zur Vorbeugung von Strahlenschäden oder durch Strahlung veranlasster Verletzung in einer oder mehreren Zellen umfassend:
Kontaktieren der einen oder mehreren Zellen vor einer Strahlenexposition mit einem Medikament wie in einem der Ansprüche 1 bis 9 definiert.

13. *In vitro*-Verfahren zur Behandlung von Strahlenschäden oder durch Strahlung veranlasster Verletzung in einer oder mehreren Zellen umfassend: Kontaktieren der einen oder mehreren Zellen mit einem Medikament wie in einem der Ansprüche 1 bis 9 definiert.

14. *In vitro*-Verfahren gemäß Anspruch 12 oder 13, wobei die eine oder mehreren Zellen nicht-kanzeröse Zellen, bevorzugt Knochenmarkzellen, sind.

## Revendications

1. Utilisation d'un composé de glycérol trisubstitué selon la formule (I) 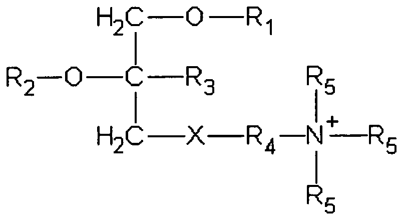 ou d'un énantiomère ou diastéréomère ou d'un sel pharmaceutiquement acceptable de ceux-ci et d'au moins un excipient pharmaceutiquement acceptable pour la fabrication d'un médicament pour la prévention et/ou le traitement de lésions ou blessures par rayonnement, où
X est sélectionné dans le groupe constitué par le phosphate et le sulfate ; R₁ est sélectionné dans le groupe constitué par l'alkyle de C₁₆-C₂₀ ;
R₂ est sélectionné dans le groupe constitué par l'alkyle de C₁-C₃ et l'hydroxyalkyle de C₁-C₃ ;
R₃ est sélectionné dans le groupe constitué par l'hydrogène et l'alkyle de C₁-C₃ ;
R₄ est sélectionné dans le groupe constitué par l'alkyle de C₁-C₃ et le cycloalkyle de C₃-C₆ ; et
R₅ est sélectionné dans le groupe constitué par l'hydrogène et le méthyle.

2. L'utilisation selon la revendication 1, où X est du phosphate, R₁ est -(CH₂)₁₇-CH₃, R₂ est CH₃, R₃ est H, R₄ est -(CH₂)₂-, et R₅ est CH₃.

3. L'utilisation selon la revendication 1 ou 2, où le médicament est une forme galénique destinée à être administrée oralement.

4. L'utilisation selon la revendication 3, où la forme galénique est une forme galénique solide.

5. L'utilisation selon la revendication 4, où la forme galénique est sélectionnée dans le groupe constitué par les comprimés, les pilules, les capsules et les granules.

6. L'utilisation selon l'une quelconque des revendications 1 à 5, où la quantité du composé de glycérol trisubstitué dans le médicament est comprise dans la plage de 30 à 250 mg, de préférence dans la plage de 50 à 150 mg.

7. L'utilisation selon l'une quelconque des revendications 1 à 6, où la dose journalière du composé de glycérol trisubstitué est comprise dans la plage de 50 à 350 mg.

8. L'utilisation selon l'une quelconque des revendications 1 à 7, où les lésions ou blessures par rayonnement sont causées par un rayonnement ionisant, le rayonnement ionisant étant de préférence sélectionné dans le groupe constitué par le rayonnement neutronique, le rayonnement alpha, le rayonnement béta, les rayons gamma, et les rayons X.

9. L'utilisation selon l'une quelconque des revendications 1 à 8, où les lésions ou blessures par rayonnement sont associées à une thérapie de cancer, de préférence à une greffe de moelle osseuse pendant une thérapie de cancer.

10. Composé de glycérol trisubstitué tel que défini dans l'une quelconque des revendications 1 à 9, destiné à être utilisé dans la prévention et/ou le traitement de lésions ou blessures par rayonnement.

11. Le composé de glycérol trisubstitué selon la revendication 10, où les lésions ou blessures par rayonnement sont associées à une thérapie de cancer ou à une greffe de moelle osseuse pendant une thérapie de cancer.

12. Procédé *in vitro* pour la prévention de lésions ou blessures par rayonnement dans une ou plusieurs cellules, comprenant : la mise en contact de l'une ou des plusieurs cellules avant une exposition au rayonnement avec un médicament tel que défini dans l'une quelconque des revendications 1 à 9.

13. Procédé *in vitro* pour le traitement de lésions ou blessures par rayonnement dans une ou plusieurs cellules, comprenant : la mise en contact de l'une ou des plusieurs cellules avec un médicament tel que défini dans l'une quelconque des revendications 1 à 9.

14. Le procédé *in vitro* selon la revendication 12 ou 13, où l'une ou les plusieurs cellules sont des cellules non cancéreuses, de préférence des cellules de moelle osseuse.
